# EUROPEAN PATENT APPLICATION

(11) **EP 1 288 226 A1**
(43) Date of publication of application: **05.03.2003**
(21) Application number: 01203325.4
(22) Date of filing: 03.09.2001
(51) Int. Cl.: C07K 14/47, C07K 14/705, A61P 25/00, A61K 38/17

(54) **Modification of the expression levels of Toll-like receptor familiy members for influencing neurodegeneration and neuroprotection in the human central nervous system**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: van Noort, Johannes Maria, 2332 AT Leiden (NL)
(74) Representative: Prins, Adrianus Willem

(57) **Abstract**

The invention provides a method for modifying the expression of at least one Toll-like receptor (TLR) in cells of the human central nervous system (CNS) comprising contacting said cells with a TLR-expression modifying agent selected from the group consisting of substances that are endogenous to the human CNS, and parts or variants thereof that are capable of altering the expression of a TLR in said cells. The invention also provides a method for retarding or inhibiting a neurodegenerative process and/or stimulating a neuroprotective process in a human being afflicted by a neurodegenerative disorder, comprising administering to said human being a TLR-expression modifying agent selected from the group consisting of substances that are endogenous to the human CNS, and parts or variants thereof that are capable of altering the expression of a TLR in cells of said CNS.

## Description

### Field of the invention

The present invention relates to methods and means which influence the function of Toll-like receptors in the human central nervous system and thereby affect neurodegenerative or neuroprotective processes. More in particular, the invention relates to the therapeutic use of endogenous central nervous system-derived molecules, specific parts or variants thereof, to influence the expression and the function of Toll-like receptors in the human central nervous system. Using such molecules either directly or indirectly, in different compositions and/or combinations, inflammatory or otherwise neurodegenerative processes can be inhibited, and neuroprotective regenerative processes can be stimulated for therapeutic approaches to central nervous system disorders in humans.

### Background of the invention

Evidence has been provided that Toll-like receptors (TLR) play a key role in mammals in the control of innate immune responses to pathogens (Medzhitov et al., 1997, Anderson, 2000; Imler and Hoffmann, 2001). TLR have been shown to recognize a wide variety of pathogen-associated molecular patterns (PAMP) and they regulate production of inflammatory mediators and other cellular functions associated with the first line of defense against pathogens. TLR are transmembrane proteins with extracellular leucine-rich repeat domains, and cytoplasmic signalling domains that are similar to the cytoplasmic domain of the interleukin-1 receptor (IL-1R) (Hardiman et al, WO 98/50547). Both the IL-1R and TLR induce signal transduction pathways leading predominantly to activation of the transcription factor NF-κB (Zhang and Ghosh, 2001; Shuto et al., 2001; Arbibe et al., 2000; Musikacharoen et al., 2001), a key regulator of inflammatory responses (May and Ghosh, 1998). Through this pathway, and others, engagement of TLR regulates the release of mediators such as TNF-α, IL-1β and nitric oxide, and the expression of co-stimulatory molecules. It is likely, however, that TLR control yet other functions in different cell types that are still undisclosed.

In the human genome, ten TLR family members have been identified to date. The precise function of most of them, however, is still largely unknown. TLR expression and functioning is the subject of intense current research, that is predominantly focussed on the role of TLR in lymphoid cells and in immunological and inflammatory processes. TLR1 is expressed in lymphoid organs in monocytes, polymorphonuclear leukocytes, T cells, B cells and NK cells (Muzio et al., 2000). TLR 2, TLR4 and TLR5 are widely expressed in myelomonocytic elements. In contrast, TLR3 has been suggested to be uniquely restricted to dendritic cells and TLR3 has not been found to be expressed on other cell types as yet (Muzio et al., 2000).

Data on ligand recognition by TLR are rapidly accumulating (recently reviewed by Imler and Hoffmann, 2001). Together, accumulated data have provided evidence for recognition by TLR of pathogen-associated molecules (pathogen-associated molecular patterns, or PAMP). TLR4 for instance plays an important role in recognition of lipopolysaccharides (LPS), TLR2 in the recognition of fungal, gram-positive and myobacterial components, and TLR5 in the recognition of bacterial flagellin (Hayashi et al., 2001). TLR9 has recently been found associated with the cellular response against bacterial CpG DNA (Hemmi et al., 2000). Evidence has been reported that TLR2 and TLR4 recognize mammalian heat shock protein 65 (Ohashi et al., 2000; Vabulas et al., 2001), a stress protein that is induced in many different types of cells by invasion of pathogens into these cells, or by pathogen-related tissue damage. While heat shock protein 65 (hsp65) is thus expressed at high levels in many types of infected cells, it is of crucial importance to note that all bacteria also express hsp65 homologs. These bacterial or fungal homologs routinely share 50% or more sequence identity to mammalian hsp65 (van der Zee et al., 1998). Thus, while hsp65 is also an endogenous molecule in mammalian cells, it is in fact structurally very similar to bacterial hsp65 and should therefore be regarded as a variant of another pathogen-associated molecule.

Interestingly, several TLR family members including TLR2 and TLR6 appear to co-operate in the recognition of PAMP in macrophages (Ozinsky et al., 2000; Bulut et al., 2001). For example, the TLR2-mediated response to phenol-soluble modulin is enhanced by TLR6 but inhibited by TLR1, indicating functional interactions between these receptors (Hajjar et al., 2001). Thus, the family of TLR appears to represent a combinatorial repertoire that allows specific recognition of large numbers of pathogen-derived products and that can discriminate between different classes of pathogens.

While the expression and functional roles of TLR in the lymphoid system has drawn much attention over the past few years, TLR expression in the human CNS has not yet been disclosed. Also, the ability of endogenous CNS-derived proteins, especially those that accumulate during neurodegenerative disease, to influence TLR functions has not yet been disclosed.

### Summary of the invention

The current invention provides data to show that in the human CNS, TLR1, TLR2, TLR3, TLR4, TLR5, TLR6, TLR7 and TLR8 are all expressed on microglia and that TLR2 and TLR3 are also expressed on astrocytes and oligodendrocytes. Thus, even a TLR that has previously been suggested to be uniquely expressed in lymphoid dendritic cells, viz. TLR3, is widely expressed on all three major types of human glia cells in the CNS.

The invention further reveals that during neurodegeneration in the human CNS, expression of all TLR is dynamically regulated, in the absence of any known pathogen or other infectious component. This indicates a role for TLR in controlling neurodegeneration and neuroprotection in the absence of pathogens.

Finally, it is revealed that endogenous CNS-derived molecules including the amyloid-β-protein-derived peptide 1-42 (further referred to herein as the amyloid peptide) and the glial stress protein alpha B-crystallin strongly influence TLR expression in microglia cells. Importantly, neither the amyloid peptide nor alpha B-crystallin have known structural relationships to any pathogen-associated molecule, nor do they have any known relationship to pathogen-associated processes in the human CNS.

Thus, we disclose a surprisingly wide expression of TLR in the human CNS, their likely role in neurodegenerative and neuroprotective processes in the absence of infection and the ability of endogenous CNS-derived molecules to regulate TLR functioning the human CNS.

### Brief description of the drawings

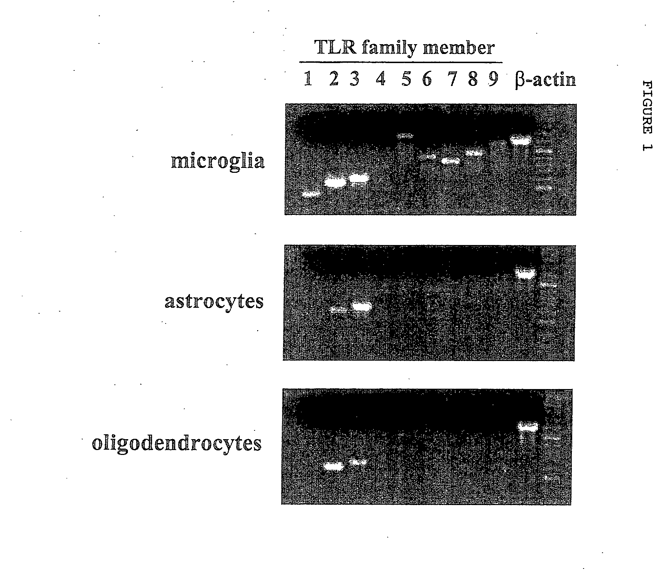

Figure 1 depicts the expression of nine TLR family members in human glia cells. Primary cultures of microglia, astrocytes and oligodendrocytes were obtained from human control white matter and TLR expression was examined by reversed-transcriptase polymerase chain reaction (RT-PCR). Amounts of cDNA used for the amplification reactions were standardized to produce identical amounts of amplicons for the housekeeping protein β-actin. The figure shows clear expression of TLR2 and TLR3 in all three types of glial cells, revealed by the appearance of the corresponding amplicons when using mRNA derived from all three types of cells. In particular microglia express TLR1, TLR2, TLR3, TLR4, TLR5, TLR6, TLR7 and TLR8 at detectable levels as well.

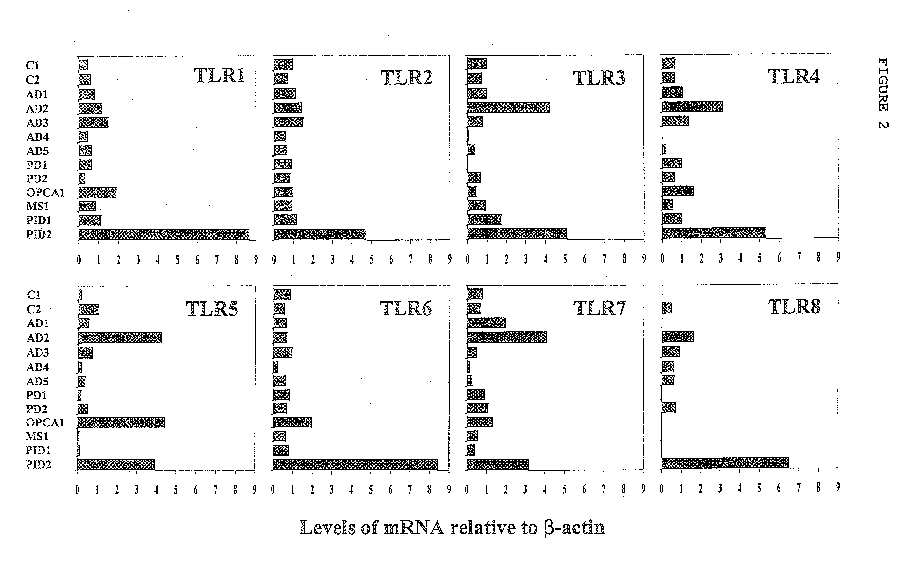

Figure 2 depicts the relative expression of nine TLR family members in primary microglia from control donors and patients with different neurodegenerative diseases. Primary cultures of microglia were obtained from white matter samples of control donors and donors that represent cases of different neurodegenerative diseases (AD = Alzheimer's disease, PD = Parkinson's disease, OPCA = olivopontocerebellar atrophy, MS = Multiple Sclerose, PID = Pick's disease). For each sample of mRNA, the level of β-actin-encoding mRNA was first determined by competitive quantitative RT-PCR. This allowed definition of standardized amount of cDNA that was subsequently used for analyzing TLR expression. Expression of mRNA encoding each TLR in each sample is expressed relative to that for β-actin.

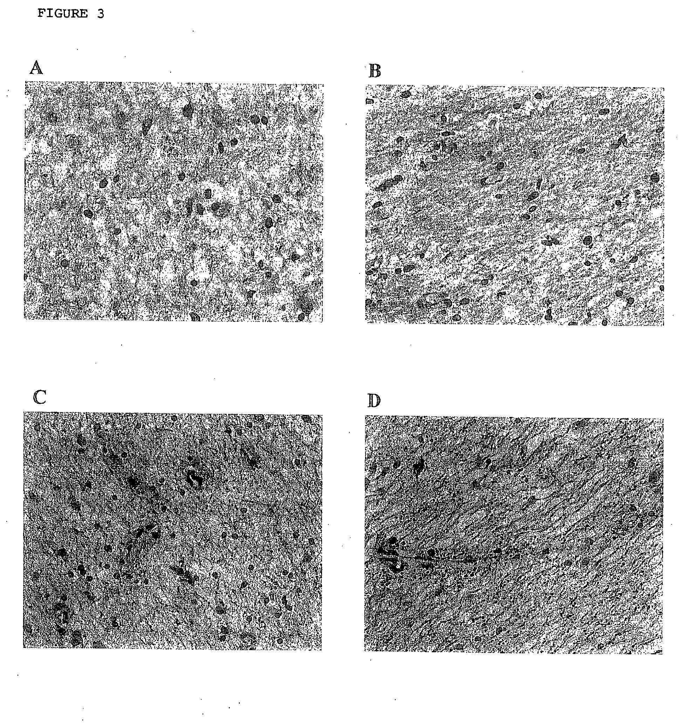

Figure 3 depicts elevated expression of TLR 3 and TLR 4 in multiple sclerosis lesions. Both healthy control white matter (A and B) and sections representing late active MS lesions (C and D) were stained for TLR3 (A and C) and TLR4 (B and D). While in healthy control white matter, TLR-containing vesicles are scarce, numbers of such vesicles are strongly increased in MS lesions, in particular in perivascular areas. Magnifications: A-D: 400x.

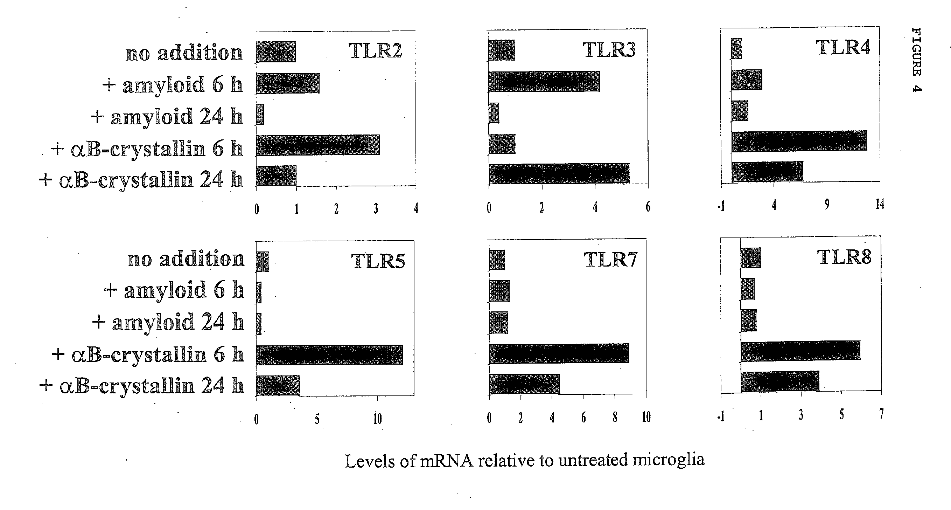

Figure 4 depicts the effect of amyloid β-protein derived peptide 1-42 and alpha B-crystallin on TLR expression levels in primary human microglia. Primary microglia from human control white matter were cultured for either 6 h or 24 h in the presence of the aggregated form of the amyloid β-protein derived peptide 1-42 or the glial stress protein alpha B-crystallin and examined by RT-PCR for levels of TLR-encoding mRNA. The figure shows that the amyloid β-protein derived peptide 1-42 has strong effects on TLR2 and TLR3 expression and that alpha B-crystallin leads to marked upregulation of TLR2, TLR3, TLR4, TLR5, TLR7 and TLR8.

### Detailed description of the invention

This invention provides a method for modifying the expression of at least one Toll-like receptor (TLR) in cells of the human central nervous system (CNS) comprising contacting said cells with a TLR-expression modifying agent selected from the group consisting of substances that are endogenous to the human CNS, and parts or variants thereof that are capable of altering the expression of a TLR in said cells.

Furthermore, the invention provides a method for retarding or inhibiting a neurodegenerative process and/or stimulating a neuroprotective process in a human being afflicted by a neurodegenerative disorder, comprising administering to said human being a TLR-expression modifying agent selected from the group consisting of substances that are endogenous to the human CNS, and parts or variants thereof that are capable of altering the expression of a TLR in cells of said CNS.

The invention also relates to the use of a TLR-expression modifying agent selected from the group consisting of substances that are endogenous to the human CNS, and parts or variants thereof that are capable of altering the expression of a TLR in cells of the human CNS, for preparing a pharmaceutical composition for retarding or inhibiting a neurodegenerative process and/or stimulating a neuroprotective process in a human being afflicted by a neurodegenerative disorder.

Further, the invention provides a pharmaceutical composition for retarding or inhibiting a neurodegenerative process and/or stimulating a neuroprotective process in a human being afflicted by a neurodegenerative disorder, comprising a TLR-expression modifying agent selected from the group consisting of substances that are endogenous to the human CNS, and parts or variants thereof that are capable of altering the expression of a TLR in cells of the human CNS, and a pharmaceutically acceptable carrier.

The TLR preferably is a member selected from the group consisting of TLR1, TLR2, TLR3, TLR4, TLR5, TLR6, TLR7 and TLR8. More particularly, the TLR is TLR2. Most preferably, the TLR is TLR3.

The cells of the CNS are human glia cells, in particular human glia cells selected from the group consisting of microglia, astrocytes and oligodendrocytes. Most preferably, the glia cells are microglia.

The substances that are endogenous to the human CNS typically are substances that accumulate, in the case of a neurodegenerative disease, in at least a part of the CNS. One preferred example of such a substance is the amyloid β-protein-derived peptide 1-42. Another preferred example of such a substance is alpha B-crystallin. The invention is however not restricted to these examples.

The TLR-expression modifying agent to be used may be one that is capable of increasing the expression level of a TLR, or one that is capable of decreasing the expression level of a TLR.

The invention is broadly applicable to any neurodegenerative disorder, including disorders like Alzheimer's disease, Parkinson's disease, Pick's disease, multiple sclerosis and stroke.

The present invention provides a surprising and novel basis for development of therapeutic approaches in human CNS disorders by targeting TLR in the human CNS. Our research established that a surprisingly broad expression of TLR occurs in human microglia, astrocytes and oligodendrocytes. We furthermore established that an altered TLR expression occurs during neurodegeneration in vivo in human brains in the absence of infectious events or pathogen-associated molecular patterns, indicative of a novel and surprising role of TLR in neurodegeneration and neuroprotection. We examined the effect of endogenous CNS-derived molecules, that have neither structural nor functional relationship to any pathogen-associated molecule or process or any other infectious event, and surprisingly established that these molecules exert influence on the levels of expression of certain TLR in human microglia. This invention concerns the utility of therapeutic molecules that do not necessarily alter TLR functioning by direct binding to TLR as agonists or antagonists, but that have their mode of action by influencing levels of expression of TLR (not excluding TLR binding by these molecules) in therapeutic treatments of neurodegenerative disorders.

While the impact of neurodegenerative diseases on human health increases, primarily by a generally increased life-expectancy, therapeutic approaches to disorders such as Alzheimer's disease, Parkinson's disease, Pick's disease, multiple sclerosis and stroke remain very limited.

Our invention can be utilized for therapeutic treatment of human CNS disorders by selectively influencing TLR expression and thereby TLR functioning in the human CNS for the purpose of inhibiting neurodegenerative processes or stimulating neuroprotective processes. Molecules can be administered that are either identical to endogenous CNS-derived proteins such as the amyloid β-protein-derived peptide 1-42 or alpha B-crystallin, or represent or contain specific parts or variants thereof. Such variants or specific parts could be designed to have optimal abilities to cross the blood-brain barrier in order to gain access to the CNS. Inside the CNS, the therapeutically administered molecules or compositions could then alter the expression and function of one specific TLR, or combinations of TLR, in that it could inhibit the contribution by certain TLR to the neurodegenerative process, or stimulate the contribution by certain TLR to neuroprotective processes to the benefit of the patient..

Our invention thus discloses the possibility to use endogenous CNS-derived molecules, or specific parts or specific variants thereof, to influence the functional expression of TLR in the human CNS. It is contemplated that in addition to the examples presently disclosed, other protein molecules (or variants or parts thereof) that are endogenous to the CNS may similarly be used to influence TLR. More specifically, it is contemplated that this applies to protein molecules that accumulate in association with neurodegenerative diseases. It is remarkable that such abnormal protein accumulation is the hallmark of several different neurodegenerative diseases (Walker and Levine, 2000; Trojanowksi and Lee, 2000). For example, apart from the amyloid β-protein derived peptide 1-42, also ubiquilin and presenillin (Mah et al., 2000) and alpha-synuclein (Hashimoto and Masliah, 1999) accumulate in the brains of Alzheimer's patients. The latter protein, alpha-synuclein, also accumulates in Lewy body disease and in Parkinson's disease (Goedert, 2001). In Parkinson's disease, also accumulation of torsin A has been reported (Shashidaran et al., 2000). It is contemplated that these abnormally accumulated proteins may well have abilities similar to the amyloid peptide or alpha B-crystallin in being able to influence TLR expression and functioning in the CNS. Thus, they could similarly be used as a basis for developing therapeutic compositions targeting TLR, as described above. Apart from the endogenous CNS-derived protein molecules themselves, also specific parts or variants of the protein sequences may be used to specifically influence TLR functioning as agonists or antagonists, that is, as molecules that bind to TLR directly, but alter or inhibit their function.

As used herein, the term "parts" relates in particular to those elements, groups or fragments of a human CNS-derived substance that in combination allow to change the expression of at least one TLR. When said human CNS-derived substance is a protein or polypeptide, "parts" would normally refer to substances containing only a part of the total amino acid sequence of the human CNS-derived substance. Said part could be a substantial part of the total amino acid sequence, or be sufficiently long to represent a peptide that is specific for the CNS-derived substance. When the human CNS-derived substance is a glycoprotein, the term "parts" would also include a form thereof which lacks part or all of the carbohydrate structures. When said human CNS-derived substance is not a polypeptide or protein but some other kind of organic compound, the term "parts" covers compounds lacking elements of the chemical structure not required for the ability to affect the expression of TLRs but containing the structural elements responsible for said ability, eg. containing the pharmacophore of the CNS-derived substance.

The term "variants" as used herein relates in particular to notional or factual derivatives of a human CNS-derived substance. Derivatives have substantial resemblance to the substance from which they are derived, but differ therefrom in at least one respect, usually by one or a few simple modifications. In the case of proteins or polypeptides, such derivatives may differ from the CNS-derived substance by substitution of one or more amino acids by others, by insertion or addition of one or more additional amino acids, or by replacement, addition or modification of one or more chemical moieties or groups. In the case of non-peptidic organic compounds, "variants" refers to notional or factual derivatives which have one or more chemical moieties of the compound replaced by others. The invention comprises the use of esters, salts, solvates, and other well known forms of derivatisation. The effect of the derivatisation may be that the ability of the compound to alter the expression of a TLR in cells of the human CNS is essentially left unchanged, or improved, or reversed, i.e. that the variant counteracts the effect on TLR expression exerted by the CNS-derived substance from which the variant is derived.

The ability of a substance (i.e. a substance endogenous to the human CNS, and parts or variants thereof) to affect the expression of at least one TLR in cells of the human CNS (i.e. to increase or suppress said expression) can be easily determined experimentally using the test described herein below.

The example studies described below disclose the existence of a broad and dynamic TLR expression in the human CNS.

It was surprisingly found that a wide range of different TLR family members are expressed in microglia. Our data (Figure 1) disclose that microglia express a wide range of TLR family members including TLR1, TLR2, TLR3, TLR4, TLR5, TLR6, TLR7 and TLR8. Especially TLR3, that has previously been suggested to be restricted to dendritic cells only (Muzio et al., 2000), is markedly expressed in microglia.

Furthermore, it was surprisingly found that astrocytes and oligodendrocytes express clear levels of TLR2 and TLR3. Constitutive high expression of TLR2 and TLR3 was found on astrocytes and oligodendrocytes (Figure 1) which is surprising since especially oligodendrocytes have no known obvious role in the control of innate immune responses or inflammation. Rather, oligodendrocytes participate in repair and regeneration in the CNS, primarily by producing the protective myelin membranes that surround and protect axons. Neuroprotective activity is also the prime role of astrocytes in the human CNS. Thus, these observations provide a clear basis for the surprising notion that TLR functioning in these types of cells will most likely be primarily associated with neuroprotective functions.

It was also surprising that neurodegeneration in vivo appeared to alter TLR expression in glia cells. Our findings as illustrated in Figure 3 prove that at least TLR3 and TLR4 are strongly increased in their expression as a result of neurodegeneration in vivo as occurs during multiple sclerosis. Cultures of primary microglia isolated from the brains of different donors with neurodegenerative diseases (Figure 2) provide further evidence for altered in vivo expression of yet more TLR, i.e. TLR1, TLR2, TLR3, TLR4, TLR5, TLR6, TLR7 and TLR8 in human microglia in neurodegenerative brains. It is surprising that this applies even to disorders without any known pathogen-associated or infectious component, such as Alzheimer's disease, Parkinson's disease and Pick's disease. Also, it is surprising that the variable levels of TLR expression were disclosed using subcortical white matter samples, therefore areas where no obvious neurodegeneration occurs in many of the cases examined. This suggests that the neurodegenerative process has effects on TLR beyond those sites that are visibly affected. The changes in TLR expression are therefore associated with -and must play a role in- either diffuse neurodegeneration in these brains, or with neuroprotective processes, but always in the absence of obvious infectious events.

It was furthermore surprisingly found that TLR expression in microglia cells may be influenced by endogenous CNS-derived molecules. We disclose that the aggregated form of the amyloid β protein-derived peptide 1-42 as well as the glia stress protein alpha B-crystallin both have strong effects on levels of TLR expression in primary human microglia within a period of 1 day (Figure 4). Amyloid β protein-derived peptide 1-42 induces elevated expression of TLR2 and TLR3, while not affecting the other TLR. In contrast, alpha B-crystallin has an even broader effect and induces markedly elevated (up to 13-fold higher) expression of TLR2, TLR3, TLR4, TLR5, TLR7 and TLR8. Both molecules exert their stimulatory effects on levels of TLR-encoding mRNA in a matter of hours.

This invention teaches the potential of endogenous CNS-derived molecules, whose expression and function in the human CNS have no known relationship to invading pathogens, and that also have no known structural similarity to pathogen-associated molecular patterns, to influence TLR functioning.

The invention will be elucidated further by the following description of example studies. It is noted, however, that this part only serves to illustrate the invention and not to define or limit its scope.

### Materials and methods used in example studies

### Tissue specimens

Human post-mortem brain tissues were obtained from control donors and patients with different neurodegenerative diseases. Characteristics of the donors are listed below in Table 1. From all donors, subcortical white matter or corpus callosum was used as a source of glia cells.

**Table 1:**

| **Brain donors** | | | | |
|---|---|---|---|---|
| donor code | sex | age | PM¹ delay | disease status |
| S01-098 | f | 96 | 05:30 | Control |
| S01-092 | m | 87 | 08:15 | Control |
| S01-009 | f | 83 | 03:30 | Alzheimer's Disease |
| S00-305 | f | 84 | 05:15 | Alzheimer's Disease |
| S00-284 | f | 87 | 03:55 | Alzheimer's Disease |
| S01-032 | f | 88 | 12:15 | Alzheimer's Disease |
| S01-030 | f | 77 | 04:40 | Alzheimer's Disease |
| S01-074 | f | 76 | 04:05 | Parkinson's disease |
| S01-095 | f | 63 | 10:55 | Parkinson's disease |
| S01-076 | f | 50 | 14:35 | olivopontocerebellar atrophy |
| S01-090 | m | 65 | 10:35 | Multiple Sclerose |
| S01-100 | m | 88 | 05.00 | Pick's disease |
| S01-156 | m | 69 | 04:55 | Pick's disease |
| ¹ PM: post-mortem delay | | | | |

### Isolation and culture of human glia cells

Primary human microglia, oligodendrocytes and astrocytes used in this study were isolated from the human white matter samples obtained from the patients listed in Table 1, as previously described (Bajramovic et al., 2000a and 2000b; De Groot et al., 2000). Briefly, brain tissues dissected from corpus callosum or subcortical white matter were collected and blood vessels were removed. After a 20-min digestion in 0.25 % (w/v) trypsine (Sigma, St. Louis, MO) and 0.1 mg/ml DNAse (Boehringer Mannheim, Mannheim, Germany) the cell suspension was gently triturated and washed with DMEM/HAM-F10 medium containing 10 % (v/v) FCS and antibiotic supplements. After passage through a 100-µm filter, myelin was removed by Percoll gradient centrifugation. Erythrocytes were lysed by a 15-min incubation on ice with 155 mM NH₄Cl, 1 mM KHCO₃, 0.2 % (w/v) BSA.

Primary cultures of microglia, astrocytes and oligodendrocytes were set up in DMEM/HAM-F10 medium containing 10 % (v/v) FCS and antibiotic supplements. Recombinant human granulocyte-macrophage colony stimulating factor (rhGM-CSF, PeproTech Inc, USA) was added to microglial cultures every 3 days at a final concentration of 20 µg/ml. Cells were analyzed for TLR expression after 10-14 days following the start of the culture.

### Semi-quantitative RT-PCR

Total cellular RNA was isolated from human glia cells using RNAZol™B (Campro scientific, Veenendaal, The Netherlands) and 2.5 µg RNA was reverse transcribed, using reverse transcription system (Promega, Madison, USA). PCR amplifications were performed on 1 µl copy DNA and 49 µl PCR mix (40 µl milliQ + 5 µl MgCl₂ (see below for concentration) + 1 µl 10 mM dNTP mix + 1 µl 20 pmol/µl primer + 1 µl 20 pmol/µl anti sense primer and 0.2 µl taq polymerase). For β-actin, TLR1, TLR2, TLR3, TLR5, TLR6, TLR7 and TLR 9, 22 mM MgCl₂ was used, for TLR4, 15 mM MgCl₂ and for TLR8, 30 mM MgCl₂. The primers used (from 5' to 3') are listed in Table 2. Amounts of TLR-encoding mRNA for each TLR were calculated by comparing the yield of each amplicon after 40 cycles to that for β-actin, and by determining the amounts of β-actin encoding mRNA in each sample by competitive quantitative RT-PCR as described previously (Bajramovic et al., 2000a; Bajramovic et al., 2000b).

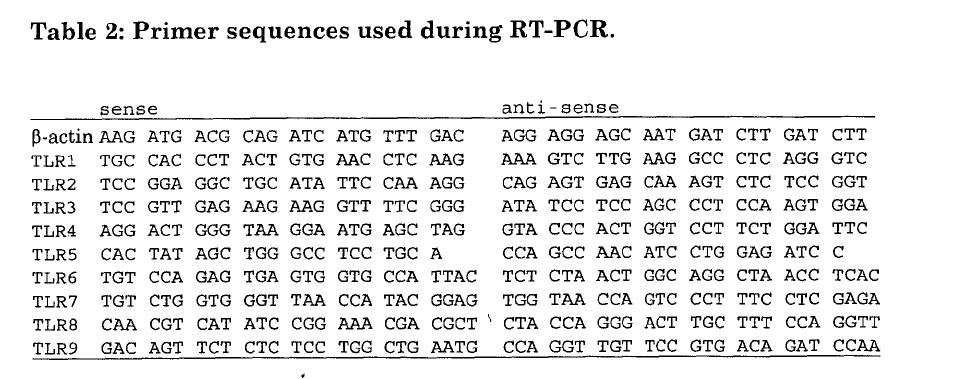

### Immunocytochemistry

Immunocytochemistry was performed on cells cultured in chamberslides. Cells were fixed with 4% formaldehyde and 0.03% H₂O₂ was added for 15 min to block endogenous peroxidase activity. Slides were incubated overnight with goat polyclonal antibodies directed against human TLR3 or TLR4 (Santa Cruz Biotechnologies, California, USA) diluted in 0.1 % (w/v) BSA in PBS plus 1% (v/v) pooled human serum. Next, the slides were rinsed with PBS + 0.05 % (w/v) Tween. As secondary antibodies, biotin-labelled donkey-anti goat antibodies were used; streptavidin FITC was subsequently used for detection of secondary antibodies.

Purity of the cultured glia cells was verified by staining for CD68 in the case of microglia, GFAP in the case of astrocytes and myelin-oligodendrocyte glycoprotein in the case of oligodendrocytes. All cultures were found to be at least 98% pure.

### Immunohistochemistry

For immunohistochemistry, formalin-fixed paraffin-embedded brain sections were used. The 5-µm sections were deparaffinized in xylene and hydrated through graded ethanol. After blocking endogenase peroxidase activity and a 15-min incubation in 1% (v/v) FCS, sections were heated for 10 min at 95°C in 10 mM sodium citrate buffer, pH 6.0. Following heat treatment, the sections were incubated overnight with anti-human TLR3 or anti-TLR4 antibodies (Santa Cruz Biotechnologies,) diluted in 0.1% (w/v) BSA in PBS plus 1% (v/v) pooled human serum. The secondary antibody used was biotinylated rabbit anti-goat Ig (DAKO, Glostrup, Denmark). After a 1 hour incubation at room temperature, slides were incubated with ABComplex/HRP (DAKO, Glostrup, Denmark) for 30 min, and subsequently for 10 min with NovaRED substrate kit (VECTOR, Burlingame, USA). Counterstaining was performed using hematoxylin. The absence of the primary antibody was used as a negative control.

### Stimulation of microglia in vitro with amyloid-β-protein-derived peptide 1-42 and alpha B-crystallin

Amyloid-β-protein-derived peptide 1-42 (Bachem, Bubendorf, Switzerland) was dissolved in water at 1 mM and kept at 37°C for 1 week to allow for aggregation to occur. The aggregated peptide was added to cultured microglia at a final concentration of 5 µM and TLR expression was determined as described above after varying periods of incubation time. As another CNS-derived stimulant, human recombinant alpha B-crystallin (prepared by TNO Prevention and Health, Leiden) was used at a final concentration of 15 µg/mL.

### Results

### Expression of TLR-encoding mRNA in primary cultures of human glia cells

As a first evaluation of TLR expression in human glia cells, the presence of TLR-encoding mRNA was examined in primary cultures of microglia, astrocytes or oligodendrocytes isolated from several different post-mortem human control brains by RT-PCR. To allow for a semi-quantitative evaluation, amounts of cDNA derived from each cell type were used that were standardized to produce the same amount of the β-actin-amplicon in all cases. The representative result for one particular control donor as represented in Fig. 1 reveals marked expression of both TLR2 and TLR3 in all three types of glia cells. In addition to TLR2 and TLR3, microglia were found to also express TLR1, TLR4, TLR6, TLR7 and TLR8 at readily detectable levels, and TLR5 at low, but still detectable levels. As a rule, TLR9 expression was undetectable in all three types of glia cells.

Next, levels of expression for each TLR family member were examined in primary cultures of microglia isolated from normal-appearing white matter samples from a variety of donors, including a new set of control subjects and donors with different neurodegenerative diseases. For microglia isolated from each donor, levels of TLR-encoding mRNA for each different TLR family member were compared to levels of β-actin-encoding mRNA, as determined by competitive quantitative RT-PCR (Bajramovic et al., 2000a; Bajramovic et al., 2000b). The result of this semi-quantitative comparison as summarized in Fig. 2 discloses remarkable differences in levels of TLR expression among different tissue samples. In microglia from both control donors analyzed, TLR1, TLR2, TLR3, TLR4, TLR5, TLR6, TLR7 and TLR8 could be detected again, consistent with the above. For each of the detected TLR family members, at least one of the donors displayed levels of expression that were markedly different from other donors, including controls. Peak expression of individual TLR was always found in cases with neurodegenerative disease rather than in control subjects. Differences in expression, however, were not always the same in donors from the same patient group.

Since the microglia examined from different donors were always isolated from subcortical white matter or corpus callosum, these tissue specimens as a rule do not include the brain sites in which tissue damage or abnormalities are visible for several of the neurodegenerative cases. For example, all Alzheimer's patients and Parkinson's disease patients suffer from a disease that affects gray matter, but does not visibly affect white matter. Also in the case of the multiple sclerosis brain, the white matter samples used as a source of microglia were normal-appearing. Still, microglia from seemingly unaffected regions of the brain do display strongly altered levels of TLR expression, despite being isolated and cultured in exactly the same way for all different donors. This provides strong indication that in a neurodegenerative human brain, widespread changes in TLR expression occur at sites distant from the local visually obvious pathological sites. It is very likely that these changes either relate to the underlying processes that cause the disease in the first place, or to widespread attempts by the brain to restore neurodegenerative damage. In other words, the changes in TLR expression in microglia must be associated either with neurodegeneration, or with neuroprotective processes.

### In vivo expression of TLR during neurodegenerative disease

To extend the data on TLR expression in primary microglia and macroglia (astrocytes and oligodendrocytes) with data on in vivo expression of TLR, expression of TLR3 and TLR4 was also evaluated in brain sections using standard immunohistochemistry. A comparison was made between healthy control white matter without any signs of neurodegeneration or damage, and brain sections from multiple sclerosis (MS) patients representing late active MS lesions. As is shown in Figs. 3A and 3B, the expression of either TLR3 or TLR4 in normal control white matter is detectable but limited, consistent with their expression in only a small minority of cultured primary microglia from healthy brains that were detected by immunocytochemical analysis (data not shown). In MS lesions, strongly elevated expression of both TLR3 and TLR4 is evident, especially in perivascular areas in MS lesions. Subcellular localization of TLR3 and TLR4 was similar in all cases, being restricted to individual vesicular structures. Surface staining of glia cells for either TLR3 or TLR4 was not observed in any tissue sample, nor in cultured primary microglia.

The data in Fig. 3 do not reveal the type of glia cell that produce TLR3- or TLR4-containing vesicles in control brain or in MS lesions. Given the expression of either TLR inside distinct but dispersed vesicles in the tissue, and given the local presence of an intricate network of microglial, astroglial and oligodendroglial processes, standard double-staining procedures at the level of light microscopy is insufficient to identify the cell type the TLR-containing vesicles belong to.

### Endogenous CNS-derived molecules strongly influence TLR expression in microglia

Primary microglia were supplied with either one of two examples of CNS-derived molecules whose expression in the human brain has been documented to be associated with neurodegenerative diseases, but that have no known structural relationship to any pathogen-associated molecule, or to any pathogen-induced process in the human CNS. The aggregated form of the amyloid β protein-derived peptide 1-42 accumulates in the brains of patients suffering from Alzheimer's disease (Clippingdale et al, 2001). The glial stress protein alpha B-crystallin accumulates in oligodendrocytes in the brains of multiple sclerosis patients, and in astrocytes in the brains of patients with a variety of neurodegenerative diseases including multiple sclerosis, Alexander's disease, Alzheimer's disease and other disorders (van Noort et al., 2000).

Addition of the aggregated form of the amyloid β protein-derived peptide 1-42 to primary microglia has a rapid mild inductive effect on levels of TLR2-encoding mRNA and equally rapid, but a stronger inductive effects on levels of TLR3-encoding mRNA (Figure 4). These effects are selective in that expression of mRNA encoding other TLR was not influenced. Alpha B-crystallin has distinctly broader effects and induces markedly elevated (up to 13-fold higher) levels of expression of mRNA encoding TLR2, TLR3, TLR4, TLR5, TLR7 and TLR8 within 6h- to 24h-incubation times. The effects of alpha B-crystallin on mRNA encoding TLR2, TLR4, TLR5, TLR7 and TLR8 were the strongest after a short 6-h period of stimulation.

Although our invention does not yet disclose via which mechanism or receptor amyloid β protein-derived peptide 1-42 or alpha B-crystallin influence TLR expression, our findings leave no doubt that both molecules do affect TLR functioning in human microglia.

### References

Anderson, K.V. 2000. Toll signaling pathways in the innate immune response. Curr Opin Immunol.12, 13-9.
Arbibe, L., Mira, J.P., Teusch, N., Kline, L., Guha, M., Mackman, N., Godowski, P.J., Ulevitch, R.J. and Knaus, U.G. 2000. Toll-like receptor 2-mediated NF-kappa B activation requires a Rac1-dependent pathway. Nat Immunol 1, 533-540.
Bajramovic, J.J., Bsibsi, M., Geutskens, S.B., Hassankhan, R., Verhulst, K.C., Stege, G.J., De Groot, C.J. and van Noort, J.M. 2000a. Differential expression of stress proteins in human adult astrocytes in response to cytokines. J Neuroimmunol 106, 14-22.
Bajramovic, J.J., Geutskens, S.B., Bsibsi, M., Boot, M., Hassankhan, R., Verhulst, K.C. and van Noort, J.M. 2000b. The stress kit: a new method based on competitive reverse transcriptase-polymerase chain reaction to quantify the expression of human alphaB-crystallin, Hsp27, and Hsp60. Cell Stress Chaperones 5, 30-35.
Bulut, Y., Faure, E., Thomas, L., Equils, O. and Arditi, M. 2001. Cooperation of Toll-Like Receptor 2 and 6 for Cellular Activation by Soluble Tuberculosis Factor and Borrelia burgdorferi Outer Surface Protein A Lipoprotein: Role of Toll-Interacting Protein and IL-1 Receptor Signaling Molecules in Toll-Like Receptor 2 Signaling. J Immunol 167, 987-994.
Clippingdale, A.B., Wade, J.D. and Barrow, C.J. (2001) The amyoid beta peptide and its role in Alzheimer's disease. J Pept Sci 7, 227-249.
De Groot, C.J., Montagne, L., Janssen, I., Ravid, R., Van, D., V and Veerhuis, R. 2000. Isolation and characterization of adult microglial cells and oligodendrocytes derived from postmortem human brain tissue. Brain Res Brain Res Protoc 5, 85-94.
Duda, J.E., Lee, V.M. and Trojanowski, J.Q. (2000) Neuropathology of synuclein aggregates. J Neurosci Res 61, 121-127.
Goedert, M (2001) Alpha-synuclein and neurodegenerative diseases. Nat Rev Neurosci 2, 492-501.
Hajjar, A.M., O'Mahony, D.S., Ozinsky, A., Underhill, D.M., Aderem, A., Klebanoff, S.J. and Wilson, C.B. 2001. Cutting edge: functional interactions between toll-like receptor (TLR) 2 and TLR1 or TLR6 in response to phenol-soluble modulin. J Immunol 166, 15-19.
Hardiman, G.T., Rock, F., Bazan, J. and Kastelein, R.A. (1998) Human Toll-like receptor proteins, related reagents and methods. International Patent Application PCT/US/98/08979; publication number WO 98/50547.
Hashimoto, M. and Masliah, E. (1999) Alpha-synuclein in Lewy body disease and Alzheimer's disease. Brain Pathol 9, 707-720.
Hayashi, F., Smith, K.D., Ozinsky, A., Hawn, T.R., Yi, E.C., Goodlett, D.R., Eng, J.K., Akira, S., Underhill, D.M. and Aderem, A. 2001. The innate immune response to bacterial flagellin is mediated by Toll-like receptor 5. Nature 410, 1099-1103.
Hemmi, H., Takeuchi, O., Kawai, T., Kaisho, T., Sato, S., Sanjo, H., Matsumoto, M., Hoshino, K., Wagner, H., Takeda, K. and Akira, S. 2000. A Toll-like receptor recognizes bacterial DNA. Nature 408, 740-745.
Imler, J.-L. and Hoffmann, J.A. 2001. Toll receptors in innate immunity. Trends Cell Biol 7, 304-311.
Mah, A.L., Perry, G., Smith, M.A. and Monteiro, M.J. (2000) Identification of ubiquillin, a novel presenilin interactor that increases presinilin protein accumulation.. J Cell Biol 151, 847-862.
May, M.J. and Ghosh, S. 1998. Signal transduction through NF-kappa B. Immunol Today 19, 80-88.
Musikacharoen, T., Matsuguchi, T., Kikuchi, T. and Yoshikai, Y. 2001. NF-kappa B and STAT5 play important roles in the regulation of mouse Toll-like receptor 2 gene expression. J Immunol 166, 4516-4524.
Medzhitov, R., Preston-Hurlburt, P. and Janeway, C.A.jr (1997) A human homologues of the drosophila Toll protein signals activation of adaptive immunity. Nature 388, 323-324.
Muzio, M., Bosisio, D., Polentarutti, N., D'amico, G., Stoppacciaro, A., Mancinelli, R., van't Veer, C., Penton-Rol, G., Ruco, L.P., Allavena, P. and Mantovani, A. 2000. Differential expression and regulation of toll-like receptors (TLR) in human leukocytes: selective expression of TLR3 in dendritic cells. J Immunol 164, 5998-6004.
Ohashi, K., Burkart, V., Flohe, S., and Kolb, H. 2000. Cutting edge: heat shock protein 60 is a putative endogenous ligand of the toll-like receptor-4 complex. J Immunol. 164, 558-61.
Ozinsky, A., Underhill, D.M., Fontenot, J.D., Hajjar, A.M., Smith, K.D., Wilson, C.B., Schroeder, L. and Aderem, A. 2000. The repertoire for pattern recognition of pathogens by the innate immune system is defined by cooperation between toll-like receptors. Proc Natl Acad Sci USA 97, 13766-13771.
Shashidharan, P., Good, P.F., Hsu, A., Perl, D.P., Brin, M.F. and Olanow, C.W. (2000) Torsin A accumulation in Lewy bodies in sporadic Parkinson's disease. Brain Res 877, 379-381.
Shuto, T., Xu, H., Wang, B., Han, J., Kai, H., Gu, X.X., Murphy, T.F., Lim, D.J. and Li, J.D. 2001. Activation of NF-kappa B by nontypeable Hemophilus influenzae is mediated by toll-like receptor 2-TAK1-depende NIK-IKKalpha /beta -Ikappa Balpha and MKK3/6-p38 MAP kinase signaling pathways in epithelial cells. 2001. Proc Natl Acad Sci USA 98, 8774-8779.
Trojanowski, J.Q. and Lee, V.M. (2000) "Fatal attractions" of proteins. A comprehensive hypothetical mechanism underlying Alzheimer's disease and other neurodegenerative disorders. Ann N Y Acad Sci 924, 62-67.
Vabulas R.M., Ahmad-Nejad, P., Da Costa, C., Miethke, T. Kirschnning, C.J., Hacker, H. and Wagner, H. 2001. Endocytosed heat shock protein 60s use TLR2 and TLR4 to activate the toll/interleukin-1 receptor signaling pathway in innate immune cells. J Biol Chem. In press
Van der Zee, R., Anderton, S.M. Prakken A.B., Paul, A.G., van Eden, W. (1998) T-cell responses to conserved bacterial heat-shock protein epitopes induces resistance in experimental autoimmunity. Sem Immunol 10, 35-41.
Van Noort, J.M., Bajramovic, J.J., Plomp, A.C. and Van Stipdonk, M.J.B. (2000) Mistaken self: a novel model that links microbial infections with myelin-directed autoimmunity in multiple sclerosis.
Walker, L.C. and LeVine, H. (2000) The cerebral proteopathies:
   neurodegenerative disorders of protein conformation and assembly. Mol Nerurobiol 21, 83-95.
Zhang, G. and Ghosh, S. 2001. Toll-like receptor-mediated NF-kappaB activation: a phylogenetically conserved paradigm in innate immunity. J Clin Invest 107, 13-19.

## Claims

1. A method for modifying the expression of at least one Toll-like receptor (TLR) in cells of the human central nervous system (CNS) comprising contacting said cells with a TLR-expression modifying agent selected from the group consisting of substances that are endogenous to the human CNS, and parts or variants thereof that are capable of altering the expression of a TLR in said cells.

2. The method of claim 1, wherein the TLR is a member selected from the group consisting of TLR1, TLR2, TLR3, TLR4, TLR5, TLR6, TLR7 and TLR8.

3. The method of claim 2, wherein the TLR is TLR2.

4. The method of claim 2, wherein the TLR is TLR3.

5. The method of any one of claims 1-4, wherein said cells are human glia cells.

6. The method of claim 5, wherein said glia cells are selected from the group consisting of microglia, astrocytes and oligodendrocytes.

7. The method of claim 6, wherein said glia cells are microglia.

8. The method of any one of claims 1-7, wherein said substances that are endogenous to the human CNS are substances that accumulate, in the case of a neurodegenerative disease, in at least a part of the CNS.

9. The method of claim 8, wherein said substance is the amyloid β-protein-derived peptide 1-42.

10. The method of claim 8, wherein said substance is alpha B-crystallin.

11. The method of any one of claims 1-10, wherein said TLR-expression modifying agent is capable of increasing the expression level of a TLR.

12. The method of any one of claims 1-10, wherein said TLR-expression modifying agent is capable of decreasing the expression level of a TLR.

13. A method for retarding or inhibiting a neurodegenerative process and/or stimulating a neuroprotective process in a human being afflicted by a neurodegenerative disorder, comprising administering to said human being a TLR-expression modifying agent selected from the group consisting of substances that are endogenous to the human CNS, and parts or variants thereof that are capable of altering the expression of a TLR in cells of said CNS.

14. The method of claim 13, wherein said substances that are endogenous to the human CNS are substances that accumulate, in the case of a neurodegenerative disease, in at least a part of the CNS.

15. The method of claim 14, wherein said substance is the amyloid β-protein-derived peptide 1-42.

16. The method of claim 14, wherein said substance is alpha B-crystallin.

17. The method of any one of claims 13-16, wherein said TLR-expression modifying agent is capable of increasing the expression level of a TLR.

18. The method of any one of claims 13-16, wherein said TLR-expression modifying agent is capable of decreasing the expression level of a TLR.

19. The method of any one of claims 13-18, wherein said neurodegenerative disorder is Alzheimer's disease.

20. The method of any one of claims 13-18, wherein said neurodegenerative disorder is Parkinson's disease.

21. The method of any one of claims 13-18, wherein said neurodegenerative disorder is Pick's disease.

22. The method of any one of claims 13-18, wherein said neurodegenerative disorder is multiple sclerosis.

23. The method of any one of claims 13-18, wherein said neurodegenerative disorder is stroke.

24. Use of a TLR-expression modifying agent selected from the group consisting of substances that are endogenous to the human CNS, and parts or variants thereof that are capable of altering the expression of a TLR in cells of the human CNS, for preparing a pharmaceutical composition for retarding or inhibiting a neurodegenerative process and/or stimulating a neuroprotective process in a human being afflicted by a neurodegenerative disorder.

25. The use of claim 24, wherein said substances that are endogenous to the human CNS are substances that accumulate, in the case of a neurodegenerative disease, in at least a part of the CNS.

26. The use of claim 25, wherein said substance is the amyloid β-protein-derived peptide 1-42.

27. The use of claim 25, wherein said substance is alpha B-crystallin.

28. The use of any one of claims 24-27, wherein said TLR-expression modifying agent is capable of increasing the expression level of a TLR.

29. The use of any one of claims 24-27, wherein said TLR-expression modifying agent is capable of decreasing the expression level of a TLR.

30. The use of any one of claims 24-29, wherein said neurodegenerative disorder is Alzheimer's disease.

31. The use of any one of claims 24-29, wherein said neurodegenerative disorder is Parkinson's disease.

32. The use of any one of claims 24-29, wherein said neurodegenerative disorder is Pick's disease.

33. The use of any one of claims 24-29, wherein said neurodegenerative disorder is multiple sclerosis.

34. The use of any one of claims 24-29, wherein said neurodegenerative disorder is stroke.

35. A pharmaceutical composition for retarding or inhibiting a neurodegenerative process and/or stimulating a neuroprotective process in a human being afflicted by a neurodegenerative disorder, comprising a TLR-expression modifying agent selected from the group consisting of substances that are endogenous to the human CNS, and parts or variants thereof that are capable of altering the expression of a TLR in cells of the human CNS, and a pharmaceutically acceptable carrier.

36. The pharmaceutical composition of claim 35, wherein said substances that are endogenous to the human CNS are substances that accumulate, in the case of a neurodegenerative disease, in at least a part of the CNS.

37. The pharmaceutical composition of claim 36, wherein said substance is the amyloid β-protein-derived peptide 1-42.

38. The pharmaceutical composition of claim 36, wherein said substance is alpha B-crystallin.

39. The pharmaceutical composition of any one of claims 35-38, wherein said TLR-expression modifying agent is capable of increasing the expression level of a TLR.

40. The pharmaceutical composition of any one of claims 35-38, wherein said TLR-expression modifying agent is capable of decreasing the expression level of a TLR.
